# EUROPEAN PATENT SPECIFICATION

(11) **EP 0 508 008 B1**
(45) Date of publication and mention of the grant of the patent: **28.08.1996**
(21) Application number: 91303113.4
(22) Date of filing: 09.04.1991
(51) Int. Cl.: C07C 5/27, C07C 11/10, C07C 11/09, B01J 21/04, B01J 23/08

(54) **Skeletal isomerization of olefins**
Verfahren zur Kettenisomerisierung von Olefinen
Procédé pour l'isomérisation du squelette d'oléfines

(43) Date of publication of application: 14.10.1992
(73) Proprietor: CHINESE PETROLEUM CORPORATION, Taipei (TW)
(72) Inventor: Lin,Chih-Cheng, Taipei (TW); Yang,Hung-Ming, Taipei (TW); Lai,Chong-Chien, Taipei (TW); Chang,Chien-Chung, Taipei (TW); Kuo,Larry L.K., Taipei (TW); Tsai,Kun-Yung, Taipei (TW)
(74) Representative: Arthur, Bryan Edward

(56) References cited:
- US-A- 2 301 342
- US-A- 3 558 733

## Description

This invention relates to the preparation of the skeletal isomerization catalyst which permits the isomerization of normal olefins to their isomers. Specifically, this invention relates to the skeletal isomerization as well as double bond isomerization of four to twenty carbon-numbers olefin. This invention more particularly relates to the conversion of linear alkenes into the branched alkenes having the same carbon numbers. The catalyst in this invention is prepared by impregnation process, then the calcination and activation steps, to form a composition of the catalyst named as SKISO-ll, an oxide of Group IIIA on the active alumina pellet.

In general, the double-bond isomerization reaction of olefin often occurs in the presence of acidic catalyst by proton transfer between olefins. However, it is not necessary to proceed the reaction at high temperature. Skeletal isomerization of olefins is known to be conducted by contacting unbranched olefins with acidic catalysts at more extreme conditions. Therefore, for skeletal isomerization of olefin, the alkyl carbonium ion which is the transition state of double-bond isomerization reaction needs to undergo a rearrangement, and subsequently a proton transfer step to form the desired products.

In the past, the skeletal isomerization catalyst were mostly prepared from the alumina support with some modifications on the surface by halogen-containing compounds, such as HBr or butyl bromide. Examples are: Sun United States Pat. 4,778,943, issued 18 Oct 1988; Sun United States Pat. 4,654,463, issued 31 Mar 1987; and Eleazar et al, United States Pat. 4,433,191, issued 21 Feb 1984. The catalyst in this invention is prepared without any modification of halide compound on the catalyst surface and can be employed to the skeletal isomerization of olefins effectively.

US 3,558,733 discloses that olefin isomerization can be improved by controlling the water content of alumina catalyst or modified aluminas such as boria-alumina and halogen treated aluminas as catalysts. US 2,301,342 discloses that olefin isomerization can be conducted by using a catalyst composed of boron oxide intimately comingled with silica or alumina in the form of a peptized gel prepared with the starting materials of hydrated aluminum sulfate and ammonium hydroxide.

The product of skeletal isomerization for C₄ olefin is isobutylene which is one of the feedstock for producing methyl tertiary butyl ether (MTBE), an ether compound with high octane number used in gasoline pool. For the skeletal isomerization of C₅ olefin, the desired product is isoamylene which is the feedstock of producing tertiary amyl methyl ether (TAME), also a high octane number compound. Hence, this invention has an implementation on improving the quality of gasoline.

It has now been discovered in this invention that the skeletal isomerization of olefins which are characterized in having from 4 to 20 carbon atoms per molecule can proceed via the action of SKISO-11 catalyst, an oxide of Group IIIA on the surface of gamma- or eta- alumina pellet. The olefin isomers of the main product will be near the equilibrium compositions, either for C₄, C₅ olefins or for other carbon numbers olefins. The activity/selectivity characteristics and stability of the catalyst employed in the process of this invention are superior to other halogenated aluminas, such as brominated or fluorinated aluminas.

Accordingly, the invention provides a catalyst for skeletal isomerization of olefins, characterised in that the catalyst consists of a support comprising pellets of crystalline alumina carrying a deposit comprising 0.01-20 wt% of a Group IIIA oxide based on the total weight of the catalyst, the catalyst containing at least 60 mole % of alumina, the said surface deposit being formed by a process which comprises impregnating the support with an acidic solution of the Group IIIA oxide in the temperature range 20°C to 90°C for 0.5 to 10 hours; calcining and activating the impregnated support at a temperature of from 300°C to 800°C for 2 to 30 hours; and cooling to room temperature.

Normal olefins are converted into iso-olefins in the presence of the SKISO-11 catalyst of this invention. The support used in the catalyst is any crystalline type of aluminas, preferably eta- or gamma-alumina with the surface areas of above 40 to about 500 m²/gram (as measured by the BET method using N₂). Other inorganic oxides may be used in the catalyst of this invention. Examples of other inorganic oxides which can be used along with alumina are magnesium oxide, calcium oxide, phosphorus oxide, tungsten oxide and palladium.

Preferably, the catalyst contains from 0.3 to about 11 wt % of a Group IIIA oxide, based on the total catalyst weight.

The preparative method for the catalyst as well as the application of the catalyst on the skeletal isomerization of normal olefins and the resulting mixture are disclosed in this invention. Typically, the catalyst of this invention can be utilized in the skeletal isomerization of olefins to convert n-butenes into isobutylene or to convert n-pentenes into isoamylenes, within which the reaction can be double bond or skeletal isomerization.

The isomerization reaction of olefins is well known to be limited by the thermodynamical equilibrium of reacting species. The skeletal isomerization catalysts described in some patents or published papers were mostly prepared from the treatment of halogen-containing compound in order to maintain the activity and the selectivity. Hence, there are some disadvantages for those catalysts, such as the investment of the facilities too high, the treatment of the process waste too difficult, etc. The objective of this invention is to propose a new catalyst for which the cost of the process investment can be reduced and the skeletal isomerization of normal olefins can easily proceed.

The preparation method of the catalyst and the reaction for C₄ or C₅ olefins are described as follows:

According to this invention, the characteristics of the preparation process for the catalyst are :
1. The active alumina pellet is impregnated in an acidic solution of Group IIIA compound in the temperature range of 20 to 90°C for 0.5 to 10 hours.
2. The solid catalyst is then calcined and activated either under air or nitrogen or hydrogen for 2 to 30 hours.
3. The calcination temperature is from 300 to 800°C and the catalyst is then cooled at room temperature to form the desired catalyst.

The feedstock of skeletal isomerization contains at least one alkene, preferably an alkene having from 4 to 8 carbon numbers per molecule. The alkene may have internal or terminal double bonds. The normal alkenes may contain other hydrocarbons having the same numbers of carbon atoms, such as alkanes. Particular feedstocks in this invention are fractions containing butenes, or mixtures of butenes with isobutylene, mixtures of pentenes with isoamylenes. Such fractions are commonly produced in petroleum refineries or petrochemical plants, for example, C₅ stream from naphtha crackers and C₄ stream from MTBE plant. The olefinic feedstocks can contain inert diluents with the content of alkenes in the range of above 10 to 95 wt % of the feed stream.

The typical example of the application of the catalyst is on the isomerization reactions, either double bond or skeletal isomerization, of the C₄ or C₅ olefins. The term C₄ olefin refers to the follow olefins: 1-butene, cis-2-butene, trans-2-butene and isobutylene. The term C₅ olefin refers to the following olefins : 1-pentene, cis-2-pentene, trans-2-pentene, 3-methyl-1-butene, 2-methyl-1-butene and 2-methyl-2-butene. The catalyst is regenerable by heating in an oxygen and nitrogen-containing gas at the temperature in the range of above 300°C to about 800°C. For maintenance of the activity of the catalyst, a hydrogen or nitrogen gas is incorporated in the reacting mixture with a molar ratio of gas to the reacting mixture of zero to about ten, preferably a molar ratio of zero to 3. The experimental apparatus and procedure are summarized below.

The said catalyst is placed in a fixed-bed reactor which has the inner diameter of 2.54 cm and the length of 30.5 cm. Some inert solid particles are placed in lower and upper ends of the reactor to support the catalyst and to make uniform distributions of the reacting stream within the reactor. The experimental apparatus also includes feed pump, heating medium, product collecting and sampling units, mass flow meter and temperature-controlled system, etc. The product is analyzed by gas chromatography, a Carlo Erba GC 6000 Vega Series unit with FID detector. The column is a capillary type.

The invention is further illustrated by the following examples which are descriptive but not limitative.

### EXAMPLE 1

According to the experimental method stated above, a better example is described as follows. A mixture which contains 94.25 wt% of 2-pentene, 1.29 wt% of isoamylene and other impurities was used as the feed. Under the reaction conditions of temperature at 350°C, 0.101 MPa (one atomsphere) and weight hourly space velocity (WHSV) at 1.0 hr⁻¹, the product distribution is obtained as 1.4 wt% of 3-methyl-1-butene, 12.8 wt% of trans-2-pentene, 5.7 wt% of cis-2-pentene, 63.3 wt% of 2-methyl-2-butene and 2-methyl-1-butene (i.e. isoamylenes), 7.8 wt% of pentane and C₄- and 8.9 wt% of C₆+ hydrocarbons. The conversion of n-pentene and the selectivity of isoamylene were estimated as 0.804 and 0.836, respectively. If pentenes in the product is normalized and compared with the equilibrium compositions of C₅ olefins which are estimated thermodynamically, a consistent result is obtained and shown in Table I.

### EXAMPLE 2

Another typical example was carried out for the skeletal isomerization of n-butene. 99.5 wt% of 1-butene was used as the feed to convert into its isomers under the reaction conditions of temperature at 450°C, 0.101 MPa (one atmosphere) and WHSV at 2.5 hr⁻¹. The product distribution was obtained as 14.8 wt% of 1-butene, 23.7 wt% of trans-2-butene, 17.2 wt% of cis-2-butene, 33.2 wt% of isobutylene, 3.4 wt% of butane and C₃- and 7.7 wt% of C₅+ hydrocarbons. The conversion of n-butene and the selectivity of isobutylene were estimated as 0.449 and 0.728, respectively. Normalizing butenes in the product, the composition is thus very consistent with the equilibrium composition of C₄ olefin which is also estimated thermodynamically. The compared result is shown in Table II.

**TABLE I**

| The comparison of C₅ olefin composition with its equilibrium composition | | |
|---|---|---|
| Component | Composition (wt%) | |
| | C₅ Product | Equilibrium |
| 1-Pentene | -- | 2.6 |
| Trans-2-pentene | 15.3 | 10.5 |
| Cis-2-pentene | 6.9 | 7.3 |
| Isoamylenes | 76.1 | 76.8 |
| 3-Methyl-1-butene | 1.7 | 2.8 |

**TABLE II**

| The comparison of C₄ olefin composition with its equilibrium composition | | |
|---|---|---|
| Component | Composition (wt%) | |
| | C₄ Product | Equilibrium |
| 1-Butene | 16.7 | 15.4 |
| Trans-2-butene | 26.4 | 26.6 |
| Cis-2-butene | 19.4 | 18.3 |
| Isobutylene | 37.5 | 39.7 |

### EXAMPLE 3

The feed is a mixture of 94.25 wt% of 2-pentene and 1.29 wt% of isoamylenes with the flow rate of 35 ml/min. The amount of the catalyst used is 5.5 grams. The operating conditions are at 0.101 MPa (one atmosphere) and different temperature, such as 200, 250, 300, 350, 400 and 450°C. The results show that the conversion of normal pentene and the selectivity of isoamylene are similar with the temperature range of 300 to 400°C. The composition of product for each condition is shown in Table III.

### EXAMPLE 4

The feed is 99.5 wt% of 1-butene with the flow rate of 100 ml/min. The operating conditions were selected as at 0.101 MPa (one atmosphere) and WHSV at 1.0 for different temperature, 250, 300, 350, 400 and 500°C. The yield of isobutylene is different for various temperature. A quick increasing of the yield of isobutylene is occurred up to 400°C reaction temperature. The composition of product for each condition is shown in Table IV.

### EXAMPLE 5

The calcination temperature of the catalyst also affects the activity of the catalyst on the skeletal isomerization due to effects on the properties of the catalyst, such as surface area and the distribution of active sites. The experimental procedure was the same as case II except that the weight hourly space velocity is at 2.5. The results are summarized in Table V.

As shown in Table V, the yield of isobutylene for each calcination temperature depicts that a better choice of calcination temperature of the catalyst is located at 500°C up.

**TABLE III**

| The product composition of C₅ skeletal isomerization for different operating temperature | | | | | | |
|---|---|---|---|---|---|---|
| Component | Composition(wt%) at Reaction Temperature(°C) | | | | | |
| | 200 | 250 | 300 | 350 | 400 | 450 |
| Pentane + C₄- | 3.2 | 4.2 | 6.3 | 7.8 | 11.7 | 21.8 |
| Trans-2-pentene | 55.9 | 39.9 | 19.0 | 12.8 | 11.7 | 10.4 |
| Cis-2-pentene | 21.6 | 16.4 | 8.2 | 5.7 | 5.5 | 5.1 |
| Isoamylenes | 16.3 | 35.1 | 57.8 | 63.3 | 60.4 | 50.0 |
| 3-Methyl-1-butene | 0.1 | 0.4 | 1.0 | 1.4 | 1.7 | 1.7 |
| C₆+ | 2.8 | 3.8 | 7.6 | 8.9 | 9.0 | 11.0 |

**TABLE IV**

| The product composition of C₄ skeletal isomerization for different operating temperature | | | | | | |
|---|---|---|---|---|---|---|
| Component | Composition(wt%) at Reaction Temperature(°C) | | | | | |
| | 250 | 300 | 350 | 400 | 450 | 500 |
| Butane + C₃- | 0.4 | 1.1 | 2.7 | 4.9 | 6.5 | 8.9 |
| 1-Butene | 15.5 | 17.8 | 16.9 | 13.3 | 12.9 | 13.6 |
| Trans-2-butene | 51.7 | 47.0 | 38.5 | 25.0 | 20.8 | 19.5 |
| Cis-2-butene | 31.8 | 30.5 | 25.9 | 17.2 | 14.8 | 14.5 |
| Isobutylene | 0.4 | 2.0 | 10.4 | 30.2 | 33.4 | 31.1 |
| C₅+ | 0.3 | 1.6 | 5.7 | 9.4 | 11.3 | 12.4 |

**TABLE V**

| The product composition of C₄ skeletal isomerization for different calcination temperature of the catalyst | | | | |
|---|---|---|---|---|
| Component | Composition(wt%) at Calcination Temperature(°C) | | | |
| | 300 | 400 | 500 | 550 |
| Butane + C₃- | 1.7 | 1.6 | 2.6 | 3.4 |
| 1-Butene | 14.9 | 15.1 | 15.4 | 14.5 |
| Trans-2-butene | 25.7 | 25.9 | 24.0 | 23.1 |
| Cis-2-butene | 19.4 | 20.5 | 17.6 | 17.4 |
| Isobutylene | 28.1 | 27.4 | 32.7 | 33.1 |
| C₅+ | 10.1 | 9.5 | 7.8 | 8.4 |

Substantially equivalent results were obtained when other operating conditions other than described above were used.

## Claims

1. A catalyst for skeletal isomerization of olefins, characterised in that the catalyst consists of a support comprising pellets of crystalline alumina carrying a deposit comprising 0.01-20 wt% of a Group IIIA oxide based on the total weight of the catalyst, the catalyst containing at least 60 mole % of alumina, the said surface deposit being formed by a process which comprises impregnating the support with an acidic solution of the Group IIIA oxide in the temperature range 20°C to 90°C for 0.5 to 10 hours; calcining and activating the impregnated support at a temperature of from 300°C to 800°C for 2 to 30 hours; and cooling to room temperature.

2. A catalyst according to Claim 1, in which the crystalline alumina support comprises gamma- or eta-alumina.

3. A catalyst according to Claim 1 or Claim 2, in which the Group IIIA oxide comprises alumina.

4. A catalyst according to any preceding claim, in which the support has a BET surface area in the range 40 to 500m²/gram.

5. A process for the skeletal isomerization and double bond isomerisation of olefin feedstocks having from 4 to 20 carbon atoms, the process being characterised by passing the feedstock at elevated temperature over a catalyst according to any preceding claim.

6. A process according to Claim 5, in which the olefin feedstock comprises C₄ olefins and the product comprises isobutylene.

7. A process according to Claim 5, in which the olefin feedstock comprises C₅ olefins and the product comprises isoamylenes.

## Patentansprüche

1. Ein Katalysator für die Kettenisomerisierung von Olefinen, dadurch gekennzeichnet, daß der Katalysator aus einem Trägermaterial besteht, das Pellets aus kristalliner Tonerde umfaßt mit einer Auflage von 0,01-20 Gew. % eines Oxids der Gruppe IIIA, ausgehend von dem Gesamtgewicht des Katalysators, wobei der Katalysator selbst mindestens 60 Mol % Tonerde enthält; die besagte Flächenauflage ist dabei durch ein Verfahren herbeigeführt, das umfaßt die Imprägnierung des Trägermaterials durch eine acidische Lösung in Form eines Oxids der Gruppe IIIA im Temperaturbereich zwischen 20°C bis 90°C über einen Zeitraum von 0,5 bis 10 Stunden; ferner die Kalzinierung und Aktivierung des imprägnierten Trägermaterials bei einer Temperatur zwischen 300°C und 800°C über einen Zeitraum von 2 bis 30 Stunden; und schließlich die Abkühlung auf Raumtemperatur.

2. Ein Katalysator gemäß Anspruch 1, bei dem das kristalline Tonerde-Trägermaterial Gamma- oder Eta-Tonerde umfaßt.

3. Ein Katalysator gemäß Anspruch 1 oder Anspruch 2, bei dem das Oxid der Gruppe IIIA Tonerde umfaßt.

4. Ein Katalysator gemäß einem der vorhergeheneden Ansprüche, bei dem das Trägermaterial einen BET-Oberflächenbereich zwischen 40 und 500 m²/g hat.

5. Ein Verfahren für die Kettenisomerisierung und Doppelbindungs-Isomerisierung von Olefin-Ausgangsmaterialien mit 4 bis 20 Kohlenstoffatomen, wobei das Verfahren dadurch gekennzeichnet ist, daß das Ausgangsmaterial bei hoher Temperatur über einen Katalysator nach einem der vorhergehenden Ansprüche geführt wird.

6. Ein Verfahren gemäß Anspruch 5, das Olefin-Ausgangsmaterial C₄-Olefine und das Produkt Isobutylen enthält.

7. Ein Verfahren gemäß Anspruch 5, bei dem das Olefin-Ausgangsmaterial C₅-Olefine und das Produkt Isoamylene enthält.

## Revendications

1. Catalyseur pour l'isomérisation squelettique d'oléfines, caractérisé en ce que le catalyseur consiste en un substrat comprenant des pastilles d'alumine cristalline portant un dépôt formé d'un oxyde du groupe IIIA de la classification périodique des éléments représentant de 0,01 à 20 % en poids du poids total du catalyseur, le catalyseur renfermant au moins 60 % en mole d'alumine, ledit dépôt de surface étant obtenu par mise en oeuvre d'un procédé qui consiste à imprégner le substrat avec une solution acide d'un oxyde du groupe IIIA dans l'intervalle de température compris entre 20 et 90°C pendant 0,5 à 10 heures, à calciner et à activer le substrat imprégné à une température comprise entre 300°C et 800°C pendant 2 à 30 heures, et à le refroidir jusqu'à la température ambiante.

2. Catalyseur selon la revendication 1, dans lequel le substrat d'alumine cristalline est l'alumine γ ou η.

3. Catalyseur selon l'une quelconque des revendications 1 ou 2, dans lequel l'oxyde du groupe IIIA est de l'alumine.

4. Catalyseur selon l'une quelconque des revendications précédentes, dans lequel le substrat présente une aire de surface B.E.T. située dans l'intervalle s'étendant de 40 à 500 m²/g.

5. Procédé pour l'isomérisation squelettique et à double liaison de substrats d'oléfines possédant de 4 à 20 atomes de carbone selon l'une quelconque des revendications précédentes, le procédé étant caractérisé en ce que l'on fait passer le substrat à température élevée sur un catalyseur.

6. Procédé selon la revendication 5, dans lequel le substrat d'oléfine comprend des oléfines C₄ et le produit comprend de l'isobutylène.

7. Procédé selon la revendication 5, dans lequel le substrat d'oléfine comprend des oléfines C₅ et le produit comprend des isoamylènes.
